# EUROPEAN PATENT APPLICATION

(11) **EP 2 295 560 A1**
(43) Date of publication of application: **16.03.2011**
(21) Application number: 09011729.2
(22) Date of filing: 14.09.2009
(51) Int. Cl.: C12N 9/78, C12N 15/55, A61K 38/50, A61P 35/00

(54) **Directed evolution of arginine deiminase for increased activity at physiological pH**

(71) Applicant: RWTH Aachen University, 52602 Aachen (DE); Jacobs University Bremen GmbH, 28759 Bremen (DE)
(72) Inventor: Schwanenberg, Ulrich, 4728 Kelmis Hergenrath (BE); Zhu, Leilei, 52074 Aachen (DE)
(74) Representative: Krauss, Jan

(57) **Abstract**

The present invention relates to modified arginine deiminases exhibiting an increased activity at a higher pH, compared to a non-modified arginine deiminase, as well as uses of these enzymes for the treatment of cancer and unwanted tumor-induced neovascularization in a cancer. The present invention further relates to a method for modifying the pH optimum of the activity of an arginine deiminase, wherein said modifying provides an increased activity of said modified arginine deiminase at a higher pH, compared to a non-modified arginine deiminase.

SEQUENCE LISTING

<110>  RWTH Aachen University
       Jacobs University Bremen

<120>  Directed evolution of arginine deiminase for increased activity
       at physiological pH

<130>  FB22040

<160>  8

<170>  PatentIn version 3.5

<210>  1
<211>  417
<212>  PRT
<213>  Pseudomonas plecoglossicida

<400>  1

Met Ser Ala Glu Lys Gln Lys Tyr Gly Val His Ser Glu Ala Gly Lys
1               5                   10                  15

Leu Arg Lys Val Met Val Cys Ala Pro Gly Leu Ala His Lys Arg Leu
            20                  25                  30

Thr Pro Ser Asn Cys Asp Glu Leu Leu Phe Asp Asp Val Ile Trp Val
        35                  40                  45

Asp Gln Ala Lys Arg Asp His Phe Asp Phe Val Thr Lys Met Arg Glu
    50                  55                  60

Arg Gly Val Asp Val Leu Glu Met His Asn Leu Leu Thr Asp Ile Val
65                  70                  75                  80

Gln Asn Pro Glu Ala Leu Lys Trp Ile Leu Asp Arg Lys Ile Thr Pro
                85                  90                  95

Asp Thr Val Gly Val Gly Leu Thr Asn Glu Val Arg Ser Trp Leu Glu
            100                 105                 110

Gly Gln Glu Pro Arg His Leu Ala Glu Phe Leu Ile Gly Gly Val Ala
        115                 120                 125

Gly Gln Asp Leu Pro Glu Ser Glu Gly Ala Ser Val Val Lys Met Tyr
    130                 135                 140

Asn Asp Tyr Leu Gly His Ser Ser Phe Ile Leu Pro Pro Leu Pro Asn
145                 150                 155                 160

Thr Gln Phe Thr Arg Asp Thr Thr Cys Trp Ile Tyr Gly Gly Val Thr
                165                 170                 175

Leu Asn Pro Met Tyr Trp Pro Ala Arg Arg Gln Glu Thr Leu Leu Thr
            180                 185                 190

Thr Ala Ile Tyr Lys Phe His Pro Glu Phe Thr Lys Ala Asp Phe Gln
        195                 200                 205

Val Trp Tyr Gly Asp Pro Asp Gln Glu His Gly Gln Ala Thr Leu Glu
    210                 215                 220

Gly Gly Asp Val Met Pro Ile Gly Lys Gly Ile Val Leu Ile Gly Met
225                 230                 235                 240

Gly Glu Arg Thr Ser Arg Gln Ala Ile Gly Gln Leu Ala Gln Asn Leu
                245                 250                 255

Phe Ala Lys Gly Ala Val Glu Gln Val Ile Val Ala Gly Leu Pro Lys
            260                 265                 270

Ser Arg Ala Ala Met His Leu Asp Thr Val Phe Ser Phe Cys Asp Arg
        275                 280                 285

Asp Leu Val Thr Val Phe Pro Glu Val Val Arg Glu Ile Val Pro Phe
    290                 295                 300

Ile Ile Arg Pro Asp Glu Ser Lys Pro Tyr Gly Met Asp Val Arg Arg
305                 310                 315                 320

Glu Asn Lys Ser Phe Ile Glu Val Val Gly Glu Gln Leu Gly Val Lys
                325                 330                 335

Leu Arg Val Val Glu Thr Gly Gly Asn Ser Phe Ala Ala Glu Arg Glu
            340                 345                 350

Gln Trp Asp Asp Gly Asn Asn Val Val Ala Leu Glu Pro Gly Val Val
        355                 360                 365

Ile Gly Tyr Asp Arg Asn Thr Tyr Thr Asn Thr Leu Leu Arg Lys Ala
    370                 375                 380

Gly Ile Glu Val Ile Thr Ile Ser Ala Gly Glu Leu Gly Arg Gly Arg
385                 390                 395                 400

Gly Gly Gly His Cys Met Thr Cys Pro Ile Val Arg Asp Pro Ile Asn
                405                 410                 415

Tyr

<210> 2
<211> 1254
<212> DNA
<213> Pseudomonas plecoglossicida

<400> 2
atgtccgctg aaaaacagaa gtacggtgtc cactccgaag caggcaagct gcgcaaggta 60

atggtctgcg ctccgggact ggcgcacaag cgcctgaccc cgagcaactg cgacgagctg 120

ctgttcgacg atgtgatctg ggtcgaccag gccaagcgcg accacttcga cttcgtcacc 180

aagatgcgcg agcgcggcgt ggatgtgctg gaaatgcata acctgctcac cgacatcgtg 240

cagaaccccg aggccctgaa gtggatcctc gaccgcaaga tcacccctga caccgtcggg 300

gtgggcctga ccaacgaagt gcgcagctgg ctggagggcc aggagccacg ccacctcgcc 360

gagttcctga tcggcggcgt ggccggccag gacctgccgg agagcgaagg tgccagcgtg 420

gtcaagatgt acaacgacta cctgggccac tccagcttca tcctgccgcc gctgcccaac 480

acccagttca cccgcgacac cacctgctgg atctacggcg gcgtgaccct caacccgatg 540

tactggccgg cgcgacgcca ggaaaccctg ctgaccaccg ccatctacaa gttccacccc 600

gagttcacca aggccgactt ccaggtctgg tacggcgacc cggaccaaga gcacggccag 660

gccaccctcg aaggcggcga cgtcatgccg atcggcaagg gcatcgtgct gatcggcatg 720

ggtgagcgca cctcgcgcca ggccatcggc caactggcac agaacctctt cgccaagggc 780

gcagtggagc aagtgatcgt cgccgggctg ccgaagtccc gtgcggccat gcacctggac 840

accgtgttca gcttctgcga ccgcgacctg gtcacggttt tcccggaagt ggtgcgcgag 900

atcgtgccgt tcatcatccg cccggacgaa agcaagccct acggcatgga cgtacgccgc 960

gagaacaagt cgttcatcga ggtggtcggc gagcagctgg gcgtcaagct gcgtgtggtc 1020

gagaccggcg gcaacagctt cgccgccgag cgcgagcagt gggatgacgg caacaacgtg 1080

gtggcgctgg agccaggtgt ggtcatcggc tacgaccgca acacctacac caataccttg 1140

ctgcgcaagg ccgggataga ggtcatcacc atcagtgccg gcgaactggg ccggggccgt 1200

ggcggcggcc actgcatgac ctgcccgatc gtgcgcgacc cgatcaacta ctaa 1254

<210> 3
<211> 29
<212> DNA
<213> Pseudomonas plecoglossicida

<400> 3
ccgcattccg ctgaaaaaca gaagtacgg 29

<210> 4
<211> 34

<212>  DNA
<213>  Pseudomonas plecoglossicida

<400>  4
cttctcgagt tagtagttga tcgggtcgcg cacg                                    34


<210>  5
<211>  33
<212>  DNA
<213>  Pseudomonas plecoglossicida

<400>  5
ggccgtggcg gcggccgttg catgacctgc ccg                                     33


<210>  6
<211>  33
<212>  DNA
<213>  Pseudomonas plecoglossicida

<400>  6
cgggcaggtc atgcaacggc cgccgccacg gcc                                     33


<210>  7
<211>  26
<212>  DNA
<213>  Pseudomonas plecoglossicida

<400>  7
tacatatgtc cgctgaaaaa cagaag                                             26


<210>  8
<211>  24
<212>  DNA
<213>  Pseudomonas plecoglossicida

<400>  8
gtgctcgagt tagtagttga tcgg                                               24

## Description

The present invention relates to modified arginine deiminases exhibiting an increased activity at a higher pH, compared to a non-modified arginine deiminase, as well as uses of these enzymes for the treatment of cancer and unwanted tumor-induced neovascularization in a cancer. The present invention further relates to a method for modifying the pH optimum of the activity of an arginine deiminase, wherein said modifying provides an increased activity of said modified arginine deiminase at a higher pH, compared to a non-modified arginine deiminase.

### Background of the invention

Arginine deiminase (ADI; EC 3.5.3.6) has been studied as a potential antitumor drug for the treatment of arginine-auxotrophic tumors, such as hepatocellular carcinomas (HCCs) and melanomas. Arginine deiminase is synthesized by various microorganisms with the highest amounts being produced by *Mycoplasma arginini.* It catalyzes the hydrolysis of L-arginine to L-citrulline.

The hepatocellular carcinoma (HCC) accounts annually for approximately 1 million new cases worldwide. In the US the incidence rate (per 100,000 persons; from 1992-2002) of HCC is 8.6 with a mortality rate of 6.5 due to low responses of hepatoma to chemotherapeutic treatments (1).

Studies with human lymphatic leukemia cell lines confirmed that ADI is a potential anti-angiogenic agent effective in the treatment of leukemia. Current research efforts are focused on ADI's in vivo inhibitory effect towards HCCs, leukemia, melanomas and human umbilical vein endothelial cells (HUVEC) (2-4), clinical trials for HCC (phase III) and melanoma (phase I/II) (3, 5), and PEG formulation of ADI to improve its efficacy as a clinical drug, including serum half-life and antigenicity (6, 7). ADI inhibited the growth of cultured leukemia cells at concentrations of 5-10 ng/ml, which were about 20-100 times lower than those of the L-asparaginase standard resulting in fewer side effects (4, 8). Arginine deprivation is speculated to be the molecular reason for ADI's inhibitory effect on human lymphatic leukemia (4). ADIs catalyze the first step of the arginine deiminase (ADI) pathway by hydrolyzing arginine to citrulline and ammonium.

Several ADI genes have been identified, purified, and characterized from bacteria, archaea and some eukaryotes excluding mammalian cells and summarized in a recent review (1). None of these ADIs is from a kinetic point of view ideally suited for *in vivo* applications in humans under physiological conditions.

Arginine deiminase from *Mycoplasma arginini* (McADI) is described, for example, by Takaku et al, Int. J: Cancer, 51:244-249 (1992), and U.S. Patent No. 5,474,928.

WO 98/51784 describes an arginine deiminase modified with polyethylene glycol, and methods of treating cancer, and methods of treating and/or inhibiting metastasis. The polyethylene glycol shall reduce the antigenicity of said arginine deiminase.

WO 1998/033519 describes an arginine deiminase from *Mycoplasma arthridis.*

WO 01/83774 describes a modified arginine deiminase of *Mycoplasma hominus* for improved manufacturing processes which is modified to be free of at least one pegylation site at or adjacent to its catalytic region. The modification can be at least one amino acid substitution at the 112, 374, 405 or 408 positions.

WO 2008/141523 describes a strain capable of producing arginine deiminase and the use thereof. In detail a method of producing arginine deiminase by fermentation of *Pseudomonas plecoglossicida* CGMCC No.2039 which is capable of producing arginine deiminase is described. By cloning the gene of arginine deiminase from the chromosomal DNA of *Pseudomonas plecoglossicida* CGMCC No.2039, the gene sequence of arginine deiminase is obtained. By fermentation culturing for 20-24 hours under aerobic condition and pH6.5-8.0 circumstance, the activity of arginine deiminase can achieve1.6U/mL fermentation liquor. The arginine deiminase produced by *Pseudomonas plecoglossicida* CGMCC No.2039 shows strong inhibition effect on liver cancer cell strain.

A main limitation of ADI from *Pseudomonas plecoglossicida* (PpADI) lies in its pH- dependent activity profile with a pH optimum at 6.5. A pH shift from 6.5 to 7.5 results in about 80% activity drop (pH of human plasma is 7.35 to 7.45).

It is therefore an object of the present invention to provide a rational mutagenesis method for pH optimized ADI enzymes that have an optimal activity, preferably at the pH of human plasma. Another object is the provision of modified pH optimized ADI enzymes, as well as respective uses of these modified enzymes for further screening and/or in cancer treatment. Other objects and advantages will become apparent from the further more detailed description of the present invention and the preferred embodiments thereof.

In the first aspect thereof, the object of the present invention is solved by a modified arginine deiminase, preferably isolated, comprising a) at least one modified amino acid in close proximity to loop 4 of said arginine deiminase and in close proximity to the active-site cysteine of said arginine deiminase, wherein said modified amino acid has a positively charged side chain at a physiological pH; and b) optionally, at least one modified amino acid in loop 1 of said arginine deiminase, wherein said modified amino acid is modified to reduce the distance with an amino acid in said loop 4 in order to favor the formation of a hydrogen bond, wherein said modified arginine deiminase exhibits an increased activity at a higher pH, compared to a non-modified arginine deiminase.

In the present invention, the inventors report the first directed ADI evolution protocol in which the pH profile of PpADI was shifted to higher pH values as further described herein representing proof that ADI properties can be tailored for demands of *in vivo* treatment of arginine-auxotrophic tumors. An emphasis is given on detailed description of directed evolution conditions (mutagenic and screening) for ensuring efficient conceptional transfers in further directed ADI evolution experiments.

Protein engineering by rational design and directed evolution offers opportunities to tailor ADI properties to physiological conditions (such as importantly the pH in human plasma or physiological saline at 7.35 to 7.45). For the proof of concept an ADI isolated from *Pseudomonas plecoglossicida* CGMCC2039 (database accession number: ABS70718) which can be functionally expressed in *E. coli* has been employed. Directed evolution approaches employ mainly *E. coli* as host due to its fast growth and high transformation efficiency which is a prerequisite for generating complex mutant libraries. Directed protein evolution has over the last decades become a versatile and successful approach for tailoring protein properties for industrial demands (mainly in chemical synthesis) and for advancing the inventors' understanding of structure-function relationships in enzymes. A directed evolution experiment comprises iterative cycles of diversity generation and functional selection for improved variants.

The crystal structures of the ADI from *Pseudomonas aeruginosa* (PaADI; PDB ID: 1RXX, 15) and *Mycoplasma arginini* (MaADI; PDB ID: 1S9R) are known. Furthermore, sequence alignments of ADIs shows that synonymous amino acid positions, and in particular the pseudosymmetrical barrel with the common arrangement ββaβ in each element of the enzyme, can be readily identified. For example, residue His404 in PpADI according to SEQ ID No. 1 corresponds to His405 in PaADI, and both *Pseudomonas* ADIs contain a His whereas both *Mycoplasma* ADIs contain an Arg at corresponding positions.

Using the BLAST algorithm, amino acids that are involved in the catalytic activity can also be readily identified as potentially preferred positions for modifications of amino acids (Stephen F. Altschul, Thomas L. Madden, Alejandro A. Schäffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402.).

The M1 variant according to the present invention (H404R) shows 40 % residual activity compared to wild type PpADI at pH 6.4 and a 1.8-fold increase in activity at pH 7.4 (see Table 2 of mutated enzymes, below). His405 in PaADI was reported (15) to share protons with Asp280 and Glu13 and to be important for modulating electrostatic environments of closely located catalytic groups. The same report postulated that H405 is likely to be protonated when the substrate arginine binds to catalytic triad (His278, Glu224, Cys406). Comparison of PaADI (pH optimum 5.6; His405) and *Mycoplasma arginini* (MaADI, pH optimum 6.5, Arg397) suggests that substitution of His by Arg might contribute to MaADI's increased pH optimum (0.9 pH units higher). A sequence alignment of four ADIs (MaADI, *Mycoplasma arthritidis* ADI, PaADI and PpADI) shows that for the synonymous amino acid positions (404 and 405), both *Pseudomonas* ADIs contain a His whereas both *Mycoplasma* ADIs contain an Arg at corresponding positions. Interestingly, both *Mycoplasma* ADIs have an approximately one pH unit higher pH optimum than the two *Pseudomonas* ADIs.

Reported ADI activities range from 0.115 to 140.3 IU/mg (1 U: One unit of ADI activity was defined as the amount of enzyme that converted 1 pmol of L-arginine into 1 pmol of L-citrulline every minute under the assay conditions) (1). PpADI is a recently isolated ADI from *Pseudomonas plecoglossicida* CGMCC2039 (7.8 U/mg) and has a pH optimum of 6.5. PpADI is a relatively slow arginine deiminase compared to *M. hominus* ADI (35 U/mg), which has the most optimal combination of physiological pH optimum and highest affinity for arginine and had been used in clinical trials (3).

Rational studies (15) postulated that position R405 might be responsible for a neutral pH optimum of *Mycoplasma arginini* ADI (McADI). *Pseudomonas aeruginosa* ADI (PaADI) shows an acidic pH optimum and contains an arginine residue in position 405 (15). Position H405 of PaADI corresponds to H404 of PpADI. The H404 position of PpADI was changed to arginine (M1) and used as starting point for directed evolution studies.

The inventors therefore improved the attractiveness of PpADI for anti-tumor applications by increasing the activity of PpADI at pH 7.4. The best mutant isolated, M2, has a specific activity of 31.3 U/mg at pH 7.4 which is close to 90% the specific activity of *Mycoplasma hominus* ADI (27) at its pH optimum.

The modified ADIs according to the invention may be modified at amino acid residues that are predicted to interfere with the activity of the ADI, in which case the goal is to create an enzyme that has a higher activity at a higher pH than does the parent enzyme. This modification can result in a variant enzyme that is related to a parent enzyme, but which is better able to hydrolyze arginine at a higher pH than is the parent enzyme. As used herein, the term "parent enzyme" means an ADI enzyme, preferably the one with the amino acid sequence of SEQ ID NO: 1.

The parent enzymes disclosed herein can be modified by the substitution of one or more residues at different, possibly selective, sites within the peptide chain. Such substitutions may be of a conservative nature, for example, where one amino acid is replaced by an amino acid of similar structure and characteristics, such as where a hydrophobic amino acid is replaced by another hydrophobic amino acid. Even more conservative would be replacement of amino acids of the same or similar size and chemical nature, such as where leucine is replaced by isoleucine. In studies of sequence variations in families of naturally occurring homologous proteins, certain amino acid substitutions are more often tolerated than others, and these are often show correlation with similarities in size, charge, polarity, and hydrophobicity between the original amino acid and its replacement, and such is the basis for defining "conservative substitutions."

Conservative substitutions are herein defined as exchanges within one of the following five groups: Group 1-small aliphatic, nonpolar or slightly polar residues (Ala, Ser, Thr, Pro, Gly); Group 2-polar, negatively charged residues and their amides (Asp, Asn, Glu, Gln) ; Group 3-polar, positively charged residues (His, Arg, Lys); Group 4-large, aliphatic, nonpolar residues (Met, Leu, Ile, Val, Cys); and Group 4-large, aromatic residues (Phe, Tyr, Trp).

Less conservative substitutions might involve the replacement of one amino acid by another that has similar characteristics but is somewhat different in size, such as replacement of an alanine by an isoleucine residue. Highly non-conservative replacements might involve substituting an acidic amino acid for one that is polar, or even for one that is basic in character. Such radical substitutions cannot, however, be dismissed as potentially ineffective since chemical effects are not totally predictable and radical substitutions might well give rise to serendipitous effects not otherwise predictable from simple chemical principles.

Of course, such substitutions may involve structures other than the common L-amino acids. Thus, D-amino acids might be substituted for the L-amino acids commonly found in the antigenic peptides of the invention and yet still be encompassed by the disclosure herein. In addition, amino acids possessing non-standard R groups (i.e., R groups other than those found in the common 20 amino acids of natural proteins) may also be used for substitution purposes to produce enzymes according to the present invention.

Other amino acid modifications involve chemial analogs of, e.g., arginine (such as for position 404) and can be selected from the following: homoarginine; L-norleucine, 5-[(aminoiminomethyl)amino]-homoarginine and NG-monomethylarginine. Similarly, chemial analogs of glutamine acid (for position 44) can be selected as following: L-glutamic acid, 4-methyl-, threo-4-methylglutamic acid; and L-threo-β-methylglutamic acid.

Additionally, specific mutations can be introduced into the arginine deiminase gene of the present invention using "site-directed mutagenesis". This is a technique standard in the art, and is conducted, e. g., using a synthetic oligonucleotide primer complementary to a single-stranded phage DNA to be mutagenized except for limited mismatching, representing the desired mutation.

Such mutations may include, for example, the deletion, insertion, or substitution of the codons expressing naturally occurring amino acids. Such mutations may confer altered protein characteristics, which may, for example, improve and/or alter the oxidative, thermal, and/or pH stability of the protein. Briefly, in this method, the synthetic oligonucleotide is used as a primer to direct synthesis of a strand complementary to the phage, and the resulting doublestranded DNA is transformed into a phage-supporting host bacterium. Cultures of the transformed bacteria are plated on agar, permitting plaque formation from single cells that harbor the phage.

If substitutions at more than one position are found to result in a enzyme with better antigenic activity as defined below, then combinations of those substitutions will be tested to determine if the combined substitutions result in additive or syngeneic effects on the activity of the enzyme (as has been done with the present sets of enzymes as shown, for example, in table 2). Preferably, at most, no more than 5 positions within the enzyme should be simultaneously substituted.

Preferred is a modified arginine deiminase according to the present invention that comprises one modified amino acid in close proximity to loop 4 of said arginine deiminase and in close proximity to the active-site cysteine of said arginine deiminase, wherein said modified amino acid has a positively charged side chain (or residue) at a physiological pH.

Further preferred is a modified arginine deiminase according to the present invention, wherein said modified amino acid is a basic amino acid, such as, for example, arginine.

Even further preferred is a modified arginine deiminase according to the present invention, comprising one modified amino acid in close proximity to loop 4 of said arginine deiminase and in close proximity to the active-site cysteine of said arginine deiminase, wherein said modified amino acid has a positively charged side chain at a physiological pH and one modified amino acid in loop 1 of said arginine deiminase, wherein said modified amino acid is modified to reduce the distance with an amino acid in said loop 4 in order to favor the formation of a hydrogen bond.

Even further preferred is a modified arginine deiminase according to the present invention, wherein said modified amino acid has an elongated side chain, such as, for example, glutamic acid.

Another aspect of the present invention relates to a modified ADI which is a fusion protein, n particular comprising fused amino acid stretches of two or more ADIs derived from different bacteria or strains of bacteria. How to create recombinant fusion proteins is known to the person of skill.

The parent enzyme of the ADI according to the present invention can be selected from any suitable ADI, preferred are parent enzymes derived from *Pseudomonas plecoglossicida* ADI, *Pseudomonas aeruginosa* ADI, *Mycoplasma arginini* ADI, *Mycoplasma arthritidis* ADI, or *Mycoplasma hominis* ADI.

Even more preferred is a modified arginine deiminase according to the present invention selected from *Pseudomonas plecoglossicida* ADI H404R or *Pseudomonas plecoglossicida* ADI H404R D44E, and *Pseudomonas plecoglossicida* ADI G113S A128T S292T H404R.

Still even more preferred is a modified arginine deiminase according to the present invention which further comprises at least one modified amino acid residue selected from position 3, 5, 30, 44, 113, 128, 147, 150, 207, 278, 291, 292, 326, 342, and 362 of *Pseudomonas plecoglossicida* ADI according to SEQ ID No. 1, or the homologous position thereof in other arginine deiminases as described both herein and in the respective literature. All these mutations improve the activity of the enzyme at a higher pH value. Furthermore, residues interacting with D44E are as follows: Arg399, Val45, Arg374, and Asp43, and residues interacting with H404R are as following: Glu13, Arg165, and Asp280. All these positions thsu are also preferred as sites for modifications according to the present invention.

Still even more preferred is a modified arginine deiminase according to the present invention wherein said arginine deiminase exhibits a decreased specific activity for a conversion of arginine to citrulline at pH 6.5, and an increased specific activity at at least one of pH 7.0; pH 7.4, or pH 7.5, and preferably exhibits a pH optimum at at least one of pH 7.0; pH 7.4, or pH 7.5. Examples for such a preferred increase at pH 7.4 compared to a pH of 6.4 can be found at a quotient of more than 0.5, preferably more than 0.8, and most preferred of more than 0.9 (see, for example, table 2).

Still even more preferred is a modified arginine deiminase according to the present invention wherein said arginine deiminase exhibits a 4-fold higher k_{cat} for a conversion of arginine to citrulline at a pH of 7.4, compared to a non-modified arginine deiminase.

Another aspect of the present invention relates to a modified ADI according to the present invention, wherein said arginine deiminase comprises at least one additional modified amino acid (modified as described above) and/or pegylation (as, for example, described in WO 98/51784), wherein said at least one modified amino acid and/or pegylation does not substantially interfere with the increased activity of said arginine deiminase at said higher pH.

Another aspect of the present invention then relates to a nucleic acid that encodes for a modified arginine deiminase according to the present invention, wherein said nucleic acid preferably is included in an expression cassette or vector.

For shifting the PpADI pH optimum, a directed evolution protocol based on an adapted citrulline screening protocol in microtiter plate format was developed and validated. Proof of concept for ADI engineering resulted in a pH optimum of pH 7.0 and increased resistance under physiological and at slight alkaline conditions: at pH 7.4, variant M2 (K5T, D44E, and H404R) is 4-fold faster than the wild type PpADI and retains about 50 % of its activity relative to pH optimum, compared to about 10 % in the case of the wild type PpADI.

Only a few manuscripts for phytases (9), xylanases (10), amylases (11-13), and an alcohol deyhdrogenase (14) reported a shift of pH optima using directed evolution algorithms. None of these directed evolution campaigns targeted a medical application and arginine deiminase has to the inventors' best knowledge not been altered in its pH activity/resistance by directed evolution. In addition, several ADI genes have been identified, purified, and characterized from bacteria, archaea and some eukaryotes excluding mammalian cells and summarized in a recent review (1). None of these ADIs is from a kinetic point of view ideally suited for *in vivo* applications in humans under physiological conditions.

In the second aspect thereof, the object of the present invention is thus solved by a method for modifying the pH optimum of the activity of an arginine deiminase, comprising the steps of a) modifying at least one amino acid in close proximity to loop 4 of said arginine deiminase and in close proximity to the active-site cysteine of said arginine deiminase, wherein said modification of said amino acid provides a positively charged side chain of said amino acid at a physiological pH; and b) optionally, modifying at least one amino acid in loop 1 of said arginine deiminase, wherein said amino acid is modified to reduce the distance with an amino acid in said loop 4 in order to favor the formation of a hydrogen bond, and wherein said modifying provides an increased activity of said modified arginine deiminase at a higher pH, compared to a non-modified arginine deiminase.

Modifying ADIs according to the invention may be achieved at amino acid residues that are predicted to interfere with the activity of the ADI, in which case the goal is to create an enzyme that has a higher activity at a higher pH than does the parent enzyme. This modification can result in a variant enzyme that is related to a parent enzyme, but which is better able to hydrolyze arginine at a higher pH than is the parent enzyme. As used herein, the term "parent enzyme" means an ADI enzyme, preferably the one with the amino acid sequence of SEQ ID NO: 1.

The parent enzymes disclosed herein can be modified by the substitution of one or more residues at different, possibly selective, sites within the peptide chain. Such substitutions may be of a conservative nature, for example, where one amino acid is replaced by an amino acid of similar structure and characteristics, such as where a hydrophobic amino acid is replaced by another hydrophobic amino acid. Even more conservative would be replacement of amino acids of the same or similar size and chemical nature, such as where leucine is replaced by isoleucine. In studies of sequence variations in families of naturally occurring homologous proteins, certain amino acid substitutions are more often tolerated than others, and these are often show correlation with similarities in size, charge, polarity, and hydrophobicity between the original amino acid and its replacement, and such is the basis for defining "conservative substitutions."

Conservative substitutions are herein defined as exchanges within one of the following five groups: Group 1-small aliphatic, nonpolar or slightly polar residues (Ala, Ser, Thr, Pro, Gly); Group 2-polar, negatively charged residues and their amides (Asp, Asn, Glu, Gln) ; Group 3-polar, positively charged residues (His, Arg, Lys); Group 4-large, aliphatic, nonpolar residues (Met, Leu, Ile, Val, Cys); and Group 4-large, aromatic residues (Phe, Tyr, Trp).

Less conservative substitutions might involve the replacement of one amino acid by another that has similar characteristics but is somewhat different in size, such as replacement of an alanine by an isoleucine residue. Highly non-conservative replacements might involve substituting an acidic amino acid for one that is polar, or even for one that is basic in character. Such radical substitutions cannot, however, be dismissed as potentially ineffective since chemical effects are not totally predictable and radical substitutions might well give rise to serendipitous effects not otherwise predictable from simple chemical principles.

Of course, such substitutions may involve structures other than the common L-amino acids. Thus, D-amino acids might be substituted for the L-amino acids commonly found in the antigenic peptides of the invention and yet still be encompassed by the disclosure herein. In addition, amino acids possessing non-standard R groups (i.e., R groups other than those found in the common 20 amino acids of natural proteins) may also be used for substitution purposes to produce enzymes according to the present invention.

Additionally, specific mutations can be introduced into the arginine deiminase gene of the present invention using "site-directed mutagenesis". This is a technique standard in the art, and is conducted, e. g., using a synthetic oligonucleotide primer complementary to a single-stranded phage DNA to be mutagenized except for limited mismatching, representing the desired mutation.

Such mutations may include, for example, the deletion, insertion, or substitution of the codons expressing naturally occurring amino acids. Such mutations may confer altered protein characteristics, which may, for example, improve and/or alter the oxidative, thermal, and/or pH stability of the protein. Briefly, in this method, the synthetic oligonucleotide is used as a primer to direct synthesis of a strand complementary to the phage, and the resulting doublestranded DNA is transformed into a phage-supporting host bacterium. Cultures of the transformed bacteria are plated on agar, permitting plaque formation from single cells that harbor the phage.

If substitutions at more than one position are found to result in a enzyme with better antigenic activity as defined below, then combinations of those substitutions will be tested to determine if the combined substitutions result in additive or syngeneic effects on the activity of the enzyme (as has been done with the present sets of enzymes as shown, for example, in table 2). Preferably, at most, no more than 5 positions within the enzyme should be simultaneously substituted.

The method can also be performed in several modification cycles, involving several mutation/modification and selection steps, in order to optimize the activity of said modified enzyme. Directed evolution allows improving all properties that can be reflected in a screening system through iterative rounds of diversity generation and screening. Small libraries of a few thousand variants are often sufficient to find improved muteins (25, 26) even without rational understanding of the targeted property. ADI properties have to date not been engineered through directed evolution despite their often poor performances under physiological conditions and their medical importance as potential anti-tumor drug. Chemically modified of ADI (e.g. through PEGylation) (1) can be used for addressing application demands. These chemical modifications (1) and ADI fusions (e.g. with human serum albumin (HSA) (28)) mainly increased ADI stability *in vivo* and will generate synergistic effects with the improvements that can be achieved through modification and/or directed evolution according to the present invention.

Preferred is a method according to the present invention, wherein said modification provides a basic amino acid, such as, for example, arginine.

Further preferred is a method according to the present invention, wherein said modification further provides an amino acid having an elongated side chain, such as, for example, glutamic acid.

Even further preferred is a method according to the present invention, wherein said arginine deiminase to be modified can be selected from *Pseudomonas plecoglossicida* ADI, *Pseudomonas aeruginosa* ADI, *Mycoplasma arginini* ADI, *Mycoplasma arthritidis* ADI, or *Mycoplasma hominis* ADI.

Still even further preferred is a method according to the present invention, wherein said modification is selected from H404R or H404R and D44E or G113S A128T S292T H404R in *Pseudomonas plecoglossicida* ADI.

Another aspect of the present invention relates to method according to the present invention, wherein said modified ADI is modified through creating a (mutated or non-mutated) fusion protein, in particular comprising fused amino acid stretches of two or more ADIs derived from different bacteria or strains of bacteria. How to create recombinant fusion proteins is known to the person of skill.

More preferred is a method according to the present invention, wherein said modification comprises at least one modification of an amino acid residue selected from position 3, 5, 30, 44, 113, 128, 147, 150, 207, 278, 291, 292, 326, 342, and 362 of *Pseudomonas plecoglossicida* ADI, or the homologous position thereof in other arginine deiminases.

Even more preferred is a method according to the present invention, wherein said arginine deiminase after modification exhibits a decreased specific activity for a conversion of arginine to citrulline at pH 6.5, and an increased specific activity at at least one of pH 7.0; pH 7.4, or pH 7.5, and preferably exhibits a pH optimum at at least one of pH 7.0; pH 7.4, or pH 7.5. Examples for such a preferred increase at pH 7.4 compared to a pH of 6.4 can be found at a quotient of more than 0.5, preferably more than 0.8, and most preferred of more than 0.9 (see, for example, table 2).

Still even more preferred is a method according to the present invention, wherein said arginine deiminase after modification exhibits a 4-fold higher k_{cat} for a conversion of arginine to citrulline at a pH of 7.4, compared to a non-modified arginine deiminase.

Still even more preferred is a method according to the present invention, wherein said method further comprises modifying of at least one additional amino acid (as above), pegylation and/or fusion to a second polypeptide, wherein said modifying of said at least one modified amino acid, pegylation and/or fusion does not substantially interfere with the increased activity of said arginine deiminase at said higher pH.

Still even more preferred is a method according to the present invention, wherein said method further comprises at least one step selected from the group consisting of mutating said arginine deiminase, such as, for example, by error-prone PCR, site directed mutagenesis, a citrulline detection assay, and a colorimetric detection assay.

Another aspect of the present invention then relates to a method for producing a modified arginine deiminase, comprising recombinant expression of a nucleic acid according to the present invention or a nucleic acid encoding for an arginine deiminase that has been modified according to the present invention, in a suitable non-human host, preferably a bacterial host. Of course, the modified arginine deiminase can also be produced (wholly or in part) by chemical synthesis.

Another aspect of the present invention then relates to a pharmaceutical composition, comprising at least one modified arginine deiminase according to the present invention, together with a pharmaceutically acceptable carrier, excipient, and/or stabilizer. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyl-dimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN^{™}, PLURONICS^{™} or polyethylene glycol (PEG).

Yet another aspect of the present invention then relates to a method for treating arginine-auxotrophic cancers, such as hepatocellular carcinomas and melanomas, comprising administering an effective amount of a modified arginine deiminase according to the present invention or a pharmaceutical composition according to the present invention to a patient in need of said treatment.

"Treatment" as used herein refers to both therapeutic treatment and prophylactic or preventative measures, wherein the object is to prevent or slow down (lessen) the targeted pathologic condition or disorder, in particular cancer. Those in need of treatment include those already with the disorder as well as those prone to have the disorder or those in whom the disorder is to be prevented. The treatment can both include adjuvant treatments and first line treatments of treatment-naive patients, and can be combined with other anti cancer strategies, such as chemotherapies. Preferably, said treatment in said patient is for the treatment of hepatocellular carcinomas and melanomas.

As described in literature, ADI inhibited the growth of cultured leukemia cells at concentrations of 5-10 ng/ml. Based on the information as provided by said literature and other clinical parameters of the patient or group of patients, as well as the disease to be treated and the state thereof, the attending physician will be able to readily determine an "effective amount" of a modified arginine deiminase according to the present invention in order to provide a treatment. It is expected that due to the improved activity of the enzyme in the physiological milieu, dosages to be administered would be advantageously lower compared to the parent enzyme, thus also limiting the potential for adverse reactions, such as, for example, imuune reactions against the ADI enzyme(s).

Similar to the above, yet another aspect of the present invention then relates to a method for treating tumor-induced neovascularization in a cancer, such as hepatocellular carcinomas and melanomas, comprising administering an effective amount of a modified arginine deiminase according to the present invention or a pharmaceutical composition according to the present invention to a patient in need of said treatment.

Similar to the above, yet another aspect of the present invention then relates to the use of a pharmaceutical composition according to the present invention, or a modified arginine deiminase according to the present invention for the treatment of arginine-auxotrophic cancers, such as hepatocellular carcinomas and melanomas. Finally, yet another aspect of the present invention then relates to the use of a pharmaceutical composition according to the present invention, or a modified arginine deiminase according to the present invention for the treatment of tumor-induced neovascularization in a cancer, such as hepatocellular carcinomas and melanomas.

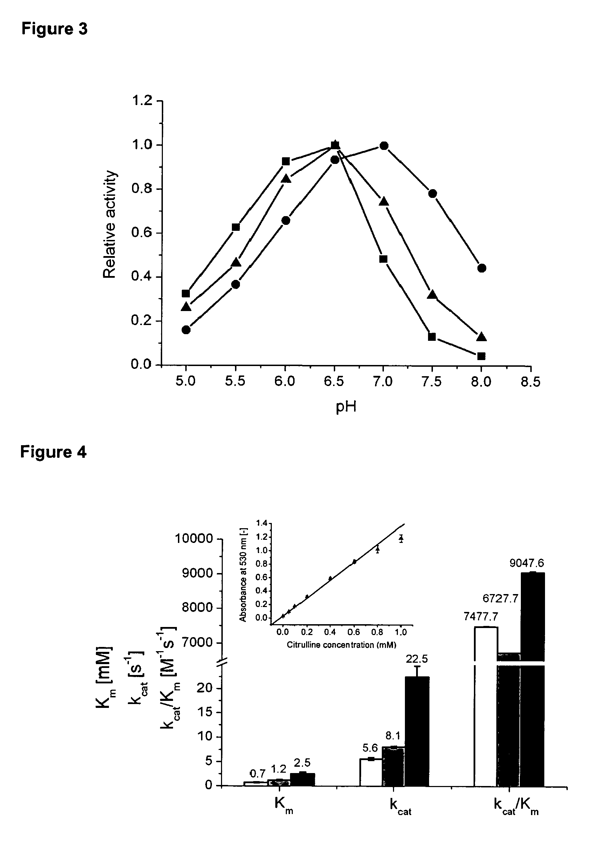

The inventors' main intention in the present invention was to establish that modification, e.g. through directed evolution, allows tailoring ADI properties directly to application demands. As an example, site directed mutagenesis of position H404R and one round of directed evolution (2400 variants screened) resulted in a triple mutant M2 (K5T D44E H404R) with 4-fold higher k_{cat} pH 7.4 (compared to wild type; Figure 4). From the two novel substitutions (K5T D44E) in M2, the substitution D44E is solely responsible for the observed k_{cat} increase (data not shown). The substitution D44E and H404R are responsible for a significant increase in (4-fold). For gaining first insights on the molecular level a model has been generated based on the PaADI crystal structures (1RXX, substrate free; 2A9G, bound arginine) (15) since a crystal structure of PpADI is not available. PpADI shares 84.9 % sequence identity with PaADI and all reported ADIs show conserved substrate binding and catalytic residues.

Residue His404 in PpADI corresponds to His405 in PaADI. The M1 variant (H404R) shows 40 % residual activity compared to wild type PpADI at pH 6.4 and a 1.8-fold increase in activity at pH 7.4 (see Table 2). His405 in PaADI was reported (15) to share protons with Asp280 and Glu13 and to be important for modulating electrostatic environments of closely located catalytic groups. The same report postulated that H405 is likely to be protonated when the substrate arginine binds to catalytic triad (His278, Glu224, Cys406). Comparison of PaADI (pH optimum 5.6; His405) and *Mycoplasma arginini* (MaADI, pH optimum 6.5, Arg397) (15) suggested that substitution of His by Arg might contribute to MaADI's increased pH optimum (0.9 pH units higher). A sequence alignment of four ADIs (MaADI, *Mycoplasma arthritidis* ADI, PaADI and PpADI) shows that for the synonymous amino acid positions (404 and 405), both *Pseudomonas* ADIs contain a His whereas both *Mycoplasma* ADIs contain an Arg at corresponding positions. Interestingly, both *Mycoplasma* ADIs have an approximately one pH unit higher pH optimum than the two *Pseudomonas* ADIs.

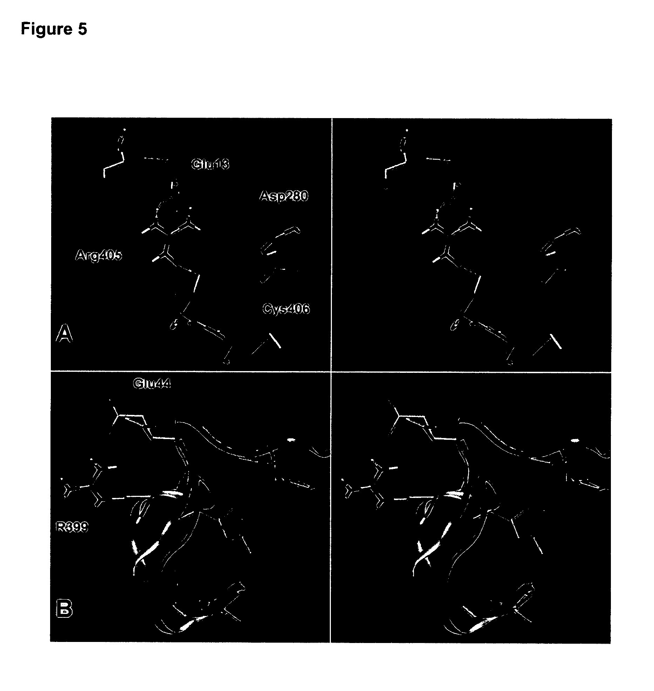

The overall pH activity profile of ADIs is modulated by the protonation state of the catalytic histidine H278 (29). A further key residue in controlling activity at acidic and neutral pH values is His405 which has been suspected by Galkin and coworkers (15) to be protonated in PaADI for efficient arginine conversion. In protonated state of the imidazolium ring, His405 can form a hydrogen bonding interaction with the oxygen of Asp280 carboxyl group. This hydrogen bond favors stabilizing interactions between Asp280 and the substrate arginine. Both effects can act concomitantly to modulate activity in both M1 and M2. In the M1 (H404R) mutant (see the model in Figure 5. A), the conformational effects due to the extended side chain of Arg likely weaken the hydrogen bond to Asp280 and thus reduces the stabilizing effect on arginine binding. As a result the activity of M1 is reduced, especially at lower pH values at which His405 is protonated. However at elevated pHs His405 becomes increasingly deprotonated, whereas Arg405 remains protonated and can therefore still stabilize Asp280 for arginine binding.

At pH 6.4, His404 in wild type PpADI and M1 (H404R) are both charged. At pH 74, His404 is partially charged while Arg404 remains protonated. Therefore, the presence of charged Arg404 in M1 positively influences the PpADI activity at higher pH.

The rationale behind the effects of the mutation D44E on the PpADI activity is more complex to extrapolate from the available ADI crystal structures. Comparative crystallographic studies have shown that the entrance of the active site is decorated by 4 loops that undergo conformational transitions upon arginine binding (15). The loops exhibit significant shifts that limit the access of water molecules to the active site. The Asp44 is located in loop 1 (residues 30-46), and upon binding of the substrate, Asp44 comes closer to the positively charged Arg399 located on loop 4 (residues 393 to 404). In the D44E mutant, the longer side chain reduce the distance with Arg399 favoring the formation of hydrogen bond (see model in Figure 5. B). The reinforced interaction between Asp44 and Arg399 might expedite conformational changes in loops, probably confining the arginine substrate in the active site and hence promote arginine conversion. Further investigated will be the influence of H404R and D44E using molecular dynamics simulations, in order to further boost the k_{cat} at pH 7.4 and reduce the Kₘ value of PpADI.

In summary, in the context of the present invention a directed evolution protocol has been developed for PpADI and validated by increasing the k_{cat} of PpADI at physiological pH (7.4). The reported screening system is the first one employed in directed ADI evolution and can be used for other ADIs and other properties such as temperature stability, salt/detergent stability or protease resistance.

The present invention shall now be described further based on the following preferred examples, nevertheless, without being limited thereto. For the purposes of the invention all references as cited herein are incorporated by reference in their entireties. The Figures and Sequences show:

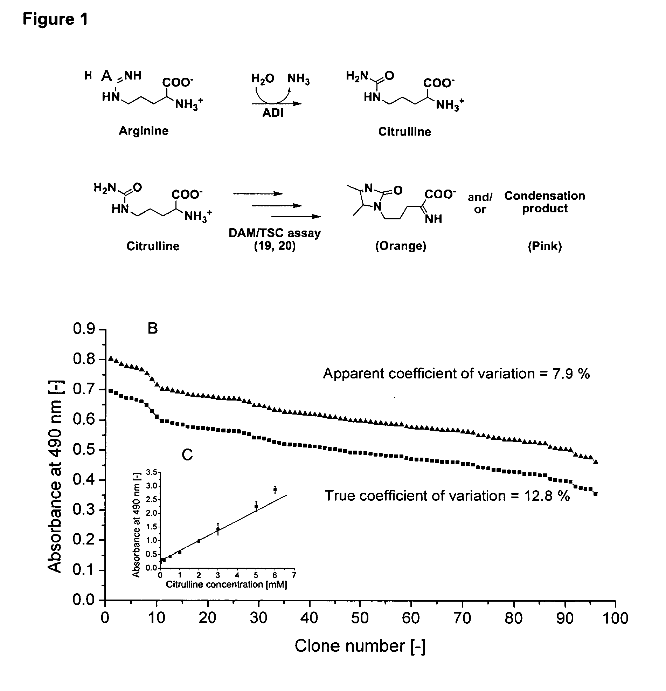

Figure 1. (A) Activity values in descending order of the ADI wild-type conversion of arginine to citrulline in a 96-well microtiter plate using the optimized assay protocol. The apparent coefficients of variation were calculated without subtracting the background and the true coefficients of variation after background subtraction. (B) Calibration curve of 96-well microtiter plate format citrulline colorimetric screening assay.

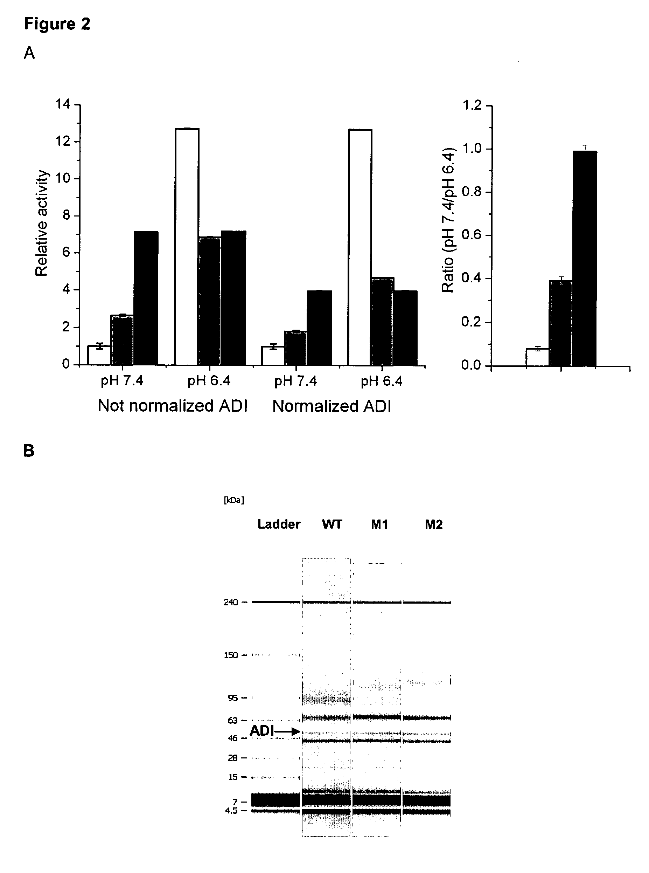

Figure 2. (A) Relative activity of ADI variants towards arginine. The relative activity is the ratio of the activity at the pH of each variant relative to the activity at pH 7.4 of wild type ADI. White: ADI wild type, grey: M1 (H404R), black: M2 (K5T D44E H404R). (B) Elution profile of wild type PpADI, variant M1 and M2 generated with Protein 230 Kit using a microfluidic device (Agilent Bioanalyzer).
Figure 3. pH profile of wild type ADI, H404R, K5T D44E H404R. The relative activity is the ratio of the catalytic activity at the pH relative to the maximum activity of each enzyme. (■) WT, (▲) M (H404R), (•) M2 (K5T D44E H404A).
Figure 4. Kinetic parameters of A01 and mutants for conversion **of** arginine to citrulline. White: ADI wild type, grey: M1 (H404R), black: M2 (K5T D44E H404R). Values reported are the average of three measurements, and average deviations from the mean values are shown. Embedded calibration curve shows linearity of citrulline calorimetric assay in 96-well PCR plate formal which was used to determine arginine conversions and kinetic constants.
Figure 5. Structural alignment of two mutant models with corresponding residues of the crystal structures of the PaADI (in orange). (A) The reference crystal structure 1RXX was used. The mutant shows a different orientation of the guanidinium group of the Arg405 with respect the Asp280. (B) The crystal structure of the inactive complex with the substrate (2A9G) was used. The extended side chain of the Glu44 improve the hydrogen bond interaction with the Arg399 improving the conformational shift of loop 1.

SEQ ID No. 1 shows the amino acid sequence of PaADI.
SEQ ID No. 2 shows the nucleic acid sequence of PaADI according to SEQ ID No. 1.
SEQ ID No. 3 to SEQ ID No. 8 show primers as used in the examples.

### EXAMPLES

### MATERIALS AND METHODS

All chemicals were of analytical-reagent grade or higher quality and were purchased from Fluka (Neu-Ulm, Germany), Sigma-Aldrich (Steinheim, Germany) and Applichem (Darm-stadt, Germany), except the resins for purification (TOSOH, Stuttgart, Germany). All enzymes were purchased from New England Biolabs (Frankfurt, Germany), Fermentas (St. Leon-Rt, Germany), and Sigma-Aldrich Chemie (Taufkirchen, Germany), unless stated otherwise.

Thermal cycler (Mastercyler gradient; Eppendorf, Hamburg, Germany) and thin-wall PCR tubes (Multi-ultra tubes; 0.2 µl; Carl Roth, Germany) were used in all PCRs. The PCR volume was always 50 µl except for Megaprimer PCR of whole plasmid (MEGAWHOP, 20 µl); larger volumes were prepared in multiple 50-µl PCRs. The amount of DNA in cloning experiments was quantified by using a NanoDrop photometer (NanoDrop Technologies, Germany).

*Pseudomonas plecoglossicida* CGMCC2039 was provided by Professor Zhihao Sun (Jiang-nan University, Wuxi, China).

### Reagents used in assays

### Acid-ferric solution

To dH₂O (600 ml), concentrated H₃PO₄ (70 ml, 85 %) and concentrated H₂SO₄ (160 ml, 90-91 %) were added slowly. After cooling to room temperature, FeCl₃-6H₂O (10 ml, 10 g/L) was added in the above solution, finally the total volume was adjusted to 1 liter with dH₂O.

### Diacetyl monoxime-thiosemicarbazide (DAM-TSC) solution

DAM solution (10 g/L) and TSC solution (0.3 g/L) were prepared separately. The two solutions were mixed (volume ratio = 1 : 1) just prior to use.

### Cloning of ADI into pET42b(+)

The arginine deiminase (ADI) gene was amplified from *Pseudomonas plecoglossicida* CGMCC2039 by colony PCR (94°C for 10 min, 1 cycle; 94°C, 30 s/ 54°C, 30 s/ 72°C, 165 s, 29 cycles; 72°C for 5 min, 1 cycle) using primer
5'-CCGCATTCCGCTGAAAAACAGAAGTACGG-3' (SEQ ID No. 3) and
5'-CTTCTCGAGTTAGTAGTTGATCGGGTCGCGCACG-3' (SEQ ID No. 4), 1.5 U of Pfu DNA polymerase, 0.20 mM of dNTP mix and a colony of *Pseudomonas plecoglossicida* CGMCC2039. Amplification product was purified by gel extraction, digested using NdeI (20 U) and XhoI (20 U) and purified using QIAquick PCR purification kit (QIAGEN, Hilden, Germany). Cloning vector pET42b(+) was digested with NdeI (20 U) and XhoI (20 U), and purified by gel extraction kit (QIAGEN, Hilden, Germany). Digested ADI gene and pET42b(+) were ligated using T4 DNA ligase (1 U) to give pET42b(+)-ADI and transformed into E. *coli* BL21-Gold (DE3). Sequence analysis of ADI gene showed that there was one synonymous mutation at amino acid position D78 (GAT to GAC) compared to the ADI-sequence of *Pseudomonas plecoglossicida* CGMCC2039 deposited in NCBI.

### Site directed mutagenesis of ADI gene at position H404

Site directed mutagenesis of ADI gene was performed according to the published method (16) on plasmid pET42b(+)-ADI. The following oligonucleotides were used for mutagenesis of H404R:
5'-GGCCGTGGCGGCGGCCGTTGCATGACCTGCCCG-3' (SEQ ID No. 5) and
5'-CGGGCAGGTCATGCAACGGCCGCCGCCACGGCC-3' ((SEQ ID No. 6) underlining indicates the substituted nucleotide). For the mutagenic PCR (First stage: 95°C for 30s, 1 cycle; 95°C, 30s/ 55°C, 1 min /72°C, 130 S, 3 cycles. Second stage: 95°C for 30 s, 1 cycle; 95°C, 30 s/ 55°C. 1 min 72°C, 130 s, 15 cycles; 60°C for 30 min, 1 cycle), 2 U of Phusion^{™} Hot Start DNA polymerase (Finnzymes, Keilaranta, Finland), 0.20 mM of dNTP mix, 25 pmol of each primer together with 100 ng of template (pET42b(+) harboring ADI gene) were used. Following the PCR, DpnI (40 U; New England Biolabs) was added, and incubated for 4 h at 37°C. The PCR products were purified by using a QIAquick PCR Purification Kit (Qiagen) and transformed into E. coli BL21-Gold (DE3) for expression.

### Construction of ADI error-prone library

The library was generated by the standard error-prone PCR (epPCR) using improved variant H404R as template. For the mutagenic PCR (95°C for 2 min, 1 cycle; 95°C, 30 s/ 55°C, 30 s/ 72°C, 30 s, 29 cycles; 72°C for 3 min, 1 cycle), 2.5 U of Taq DNA polymerase, 0.20 mM of dNTP mix, 50 ng of template (pET42b(+) harboring ADI gene H404R), 0.01-0.1 mM of MnCl₂ and 10 pmol of each primer
5'-TACATATGTCCGCTGAAAAACAGAAG-3' (SEQ ID No. 7) and
5'-GTGCTCGAGTTAGTAGTTGATCGG-3' (SEQ ID No. 8)
were used. The PCR products were purified by using a QIAquick PCR Purification Kit. The purified epPCR products were cloned into expression plasmid pET42b(+) by MEGAWHOP (17). For MEGAWHOP (60°C for 5 min, 1 cycle; 95°C for 5 min, 1 cycle; 95°C, 1 min 55°C, 1 min 68°C, 13 min 30 s, 24 cycles; 60°C for 30 min, 1 cycle), 1 U of Taq DNA polymerase, 0.1 U of Pfu DNA polymerase, 0.20 mM of dNTP mix together with 200 ng of template (pET42b(+) harboring ADI gene H404R) were used. Following the PCR, DpnI (40 U; New England Biolabs) was added, and incubated (4 h; 37°C). The MEGAWHOP products were transformed into E. *coli* BL21 -Gold (DE3) for expression and screening.

### Cultivation and expression in 96-well plates

Colonies grown on LBₖₐₙ, agar plates were transferred, by using toothpicks, into 96-well microtiter plates (flat bottom, polystyrene plates; Greiner Bio-One GmbH, Frickenhausen, Germany), containing non-inducing medium LSG (150 µl) (18) supplemented with kanamycin (50 µg/ml). After 16 h of cultivation in a microtiter plate shaker (Multitron II, Infors GmbH, Einsbach, Germany; 37°C, 900 rpm, 70 % humidity), each well was replicated using a replicator (EnzyScreen BV, Leiden, Netherlands) into a second series of 96-well microtiter plates containing 150 µL of auto-induction media LS-5052 (18) supplemented with kanamycin (50 pg/ml). The first set of plates was stored at -80°C after addition of glycerol. The clones in the second set of plates were cultivated for 12 h (Multitron II, Infors GmbH, 37°C, 900 rpm) and used for screening.

### Screening Procedure

### 96-well plate format citrulline colorimetric screening assay

A modified protocol of citrulline detection based on carbamido-diacetyl reaction (19) (see Figure 1A for assay mechanism) was used for ADI activity measurement. Screening for increased activity was carried out by measuring the activity at pH 7.4 and 6.4.

20 µl of cell culture was transferred to 96-well microtiter plate. Enzyme reaction was initiated by addition of arginine solution (100 µl, 100 mM) supplemented with cetyltrimethylammoniumbromide (CTAB, 4 mM), and incubated (20 min, 37°C). Subsequently, acid-ferric solution (60 µl) and diacetyl monoxime (DAM, 20 µl, 0.5 M) were added. The reaction mixture was further incubated (15 min, 55°C). Absorbance was measured at 492 nm using a microtiter plate reader (Tecan Sunrise, Tecan Group AG, Zurich, Switzerland).

Standard deviation measurements were performed in 96-well plate format using culture from BL21-Gold (DE3) lacking ADI and in a separate experiment containing ADI. Apparent standard deviation was based on the absolute absorbance values obtained from the ADI wild type plate. The true standard deviation was calculated by subtracting the background absorbance value of the microtiter plate lacking ADI from the apparent values.

### Cuvette format citrulline calorimetric assay

ADI activity was routinely measured using a modified protocol of the citrulline detection with DAM and TSC (20) (see Figure 1A for assay mechanism) in Eppendorf tubes. Enzyme reaction was initiated by addition of arginine solution (200 µl, 100 mM) to a 2-ml Eppendorf tube containing crude cell lysate (50 µl), and incubated (20 min, 37°C). Subsequently, acid-ferric solution (250 µl) was added to stop the enzyme reaction. After appropriate dilution with deionized water to ensure the concentration of produced citrulline is within linear detection range, the reaction mixture (400 µl) was mixed with acid-ferric solution (600 µl) and DAM-TSC solution (100 µl). The reaction mixture was further incubated (30 min, 70°C), followed by incubation in ice water to stop color development. Absorbance was measured at 530 nm using a Specord 200 (Analytik Jena AG, Jena, Germany).

### Normalization of protein expression for wild type ADI and variants

Agilent Protein 230 Kit (Agilent Protein 230 Kit, Agilent Technologies Deutschland GmbH, Böblingen, Germany) and Agilent 2100 Bioanalyzer (Agilent 2100 Bioanalyzer, Agilent Technologies Deutschland GmbH, Böblingen, Germany) were used for normalization of protein expression in crude cell extract. The protocol used was according to Agilent protein 230 Kit Guide except BSA was used as a internal standard.

### Expression of ADI in shaking flask and purification

Shaking flasks (1 liter) containing auto induction media LS-5052 (200 ml) supplemented with kanamycin (50 µg/ml) were inoculated with a 1 : 200 dilution of overnight culture (E. *coli* BL21-Gold(DE3) harboring pET42b-ADI) grown in non-inducing media LSG. After 12 h of expression, E. *coli* cells were harvested by centrifugation (Eppendorf 5810R 4°C, 3220 g, 30 min) and resuspended in phosphate buffer (20 ml, NaₓPO₄, 20 mM, pH 7.0). E. *coli* cells were subsequently lysed by using a high-pressure homogenizer (1500 bar, 2 cycles; Avestin Emulsiflex, Mannheim, Germany). The disrupted cells were centrifuged (Eppendorf 5417R, 4°C, 13000 g, 20 min) and the supernatant was further cleared by filtration through a low protein-binding filter (0.45 um; Minisart RC 25 single use syringe filter; Sartorius, Hamburg, Germany). ADI wild type and mutants (H404R and K5T D44E H404R) were subsequently purified by a column chromatography procedure: (i) The filtered cell lysates were subjected to a Super-Q anion exchange column, which had been pre-equilibrated with 50 mM phosphate buffer (NaₓPO₄, pH 7.0). 20 ml of cell lysate was loaded. ADI were eluted by a NaCl step elution in phosphate buffer (NaₓPO₄, 50 mM, pH 7.0) at a rate of 3 ml/min. (ii) The obtained ADI protein from ion exchange chromatography was subjected to a gel filtration column (Matrix: Toyoperl HW-55S, Bed volume: 33 ml, Bed height: 40 cm, Column: Omnifit).

Purified ADI was subsequently concentrated using a Amicon ultra-4 centrifugal filter device (Millipore Corporation, Billerica, U.S.A.) with a 30-kDa cut-off membrane. Total protein concentration was determined by BCA^{™} assay kit (Pierce, Born, Germany) and homogeneity of the purified sample was controlled by SDS-page electrophoresis using standard molecular biology techniques.

### Characterization of ADI wild type and mutants

### Determination of k_{cat} and Kₘ of ADI wild type and the mutants

The k_{cat} and Kₘ values were determined from initial velocity data measured as a function of substrate concentration. Enzyme reaction was carried out at 37°C in a water bath. After 10 min preincubation at 37"C, the enzyme reaction was initiated by addition of purified enzyme (50 µl, 0.2-0.5 µM) to the substrate solution (200 µl, 0.2 mM to 10 mM of arginine, 0.5 M phosphate buffer, pH 7.4) in deep well plates. The reaction mixture was incubated (37°C) and every two minutes reaction mixture (30 µl) of each well was transferred to acid-ferric solution (30 µl) to stop the enzyme reaction. The color development was subsequently performed in 96-well PCR plate. Ferric-acid solution (90 µl) and DAM-TSC solution (15 µl) were added to each well. The 96-well PCR plate was then incubated (70°C, 30 min) in thermal cycler (Eppendorf Mastercyler gradient) for color development, followed by incubation in ice water to stop color development. Absorbance was measured at 530 nm using a microtiter plate reader (SPECTROstar Omega, BMG LABTECH, Offenburg, Germany). The initial velocity data obtained were fitted to the equation v = Vₘₐₓ[S]/([S] + Kₘ) (where v is the initial velocity, Vₘₐₓ the maximum velocity, [S] the substrate concentration, and Kₘ the Michaelis constant) using GraphPad Prism software (GraphPad software, San Diego, CA, USA). The k_{cat} was calculated from the ratio of Vₘₐₓ, and enzyme concentration.

### Determination of pH profile

Enzyme reaction was carried out at 37°C in a water bath using the following substrate solution: arginine 0.1 M, pH 5-8 phosphate/acetate buffer (0.5 M phosphate, 50 mM acetate). After 10 min preincubation at 37°C, the enzyme reaction was initiated by addition of purified enzyme (50 µl, 0.2-0.5 µM) to the substrate solution (200 µl) in deep well plates. The reaction mixture was incubated (37°C, 10 min) and acid-ferric solution (250 µl) was transferred to each well to stop the enzyme reaction. The color development was subsequently performed in 96-well PCR plate. The reaction mixture (60 µl), ferric-acid solution (90 µl) and DAM-TSC solution (15 µl) were transferred to a 96-well PCR plate. The 96-well PCR plate was then incubated (70°C, 30 min) in thermal cycler (Eppendorf Mastercyler gradient) for color development, followed by incubation in ice water to stop color development. Absorbance was measured at 530 nm using a microtiter plate reader (SPECTROstar Omega, Germany).

### Molecular Modeling

Two crystal structures of PaADI (PDB codes: 1RXX, 2A9G) were used to model the mutations H405R and D44E. Only one monomeric unit of the tetrameric PaADI was used for the modeling study employing Swiss-PDB viewer (21) (www.expasy.org/spdbv). PaADI variants (H405R; D44E) were generated using the mutate command of Swiss-PDB viewer. The side chain rotamer with the best score was used for subsequent energy optimized with parameter of GROMOS96 force field for in vacuum minimization (22).

### PpADI screening system for citrulline production in microtiter plates

A modified version of the DAM-TSC assay for citrulline detection (Figure 1A) was used in microtiter plate screening. Two key performance parameters for identifying improved variants in microtiter plate based screening systems are the true standard variation and the linear detection range of the employed assay. Standard deviations have been reduced by optimizing expression and screening conditions (various auto-induction media and assay conditions such as amount of cell, conversion time and color development procedure). The preferred combination (150 µl LS5052 auto-induction media and 20 µl cell culture, 20 min conversion time and 15 min color develop time) resulted in a true standard deviation of 12.8% after subtracting the background (Figure 1 B). Screening systems with standard deviations around 10% have successfully been used in directed evolution experiments (23, 24). A wide linear detection window is important for identifying beneficial mutants in directed evolution experiments and screening systems for evolved mutants have to be constantly adapted for hitting the linear detection window. A linear detection range up to 6 mM citrulline could be detected (Figure 1 C); a concentration of up to 6 mM was detected in microtiter plate screens (Figure 1 C).

### Validation of M1 (H404R) using cuvette format citrulline calorimetric assay

Position H404 was suspected to influence AD1I pH profile by substitution to R404 (15). Compared to wild type PpADI, variant M1 (H404R) shows higher activity (1.8-fold) and improved activity ratio of pH 7.4 to 6.4 (4.7-fold). Therefore, M1 (H404R) was selected for directed evolution.

### Validation of citrulline production assay by screening error-prone mutant libraries

Various concentrations of MnCl₂ (0.01-0.10 mM) were used in epPCR library generation to adjust the ratio of active to inactive clones. A concentration of 0.01 mM MnCl₂ which generated 53% inactive mutant was selected for library generation. EpPCR mutant libraries with a ratio of 50% active and 50% inactive clones have often been used successfully in directed evolution, improving enzyme properties by gradual changes (one or two amino acids changes per round of mutagenesis and screening). A total of 9 variants (listed in Table 2) were identified from an epPCR library of 2400 mutants and used for further characterization. The most active variant was sequenced and two additional amino acid substitutions (K5T D44E) were identified.

### Characterization of PpADI variants

Variants selected from epPCR were first characterized using the crude cell extract to identify the most promising variants (Table 2). Using an activity ratio of pH 7.4 to 6.4 proved to be an effective method to eliminate expression mutants. Additionally ADI concentration in crude cell extract was determined using the Agilent Protein 230 Kit. Microfluidic elution profiles (Figure 2) semi-quantify differences in expression between the wild type PpADI and variants. Normalization of activities against the corresponding ADI concentrations reveals improvements in specific activities of ADI mutants (Table 2). M1, the starting variant for the directed evolution campaign, and M2 were selected for detailed characterization studies. M2 has as "best identified variants" (Table 2) a more than 2.5-fold higher activity ratio at pH 7.4 to pH 6.4 than M1 and a 12-fold higher activity ratio than wild type in crude cell lysates.

For kinetic characterization of ADI variants the citrulline production was maintained between 0.2-0.8 mM. The calibration curve in Figure 4 shows the linear detection range in the employed 96-well PCR plate format citrulline colorimetric assay system. Figure 4 summarizes the k_{cat} and Kₘ values of wild type PpADI, identified variants M1 (H404) and M2 (K5T D44E H404R) which were expressed in shaking flasks cultures and purified using a two step procedure (anion exchange follows by gel filtration). Both variants M1 and M2 show an increase in k_{cat} and Kₘ for citrulline production at pH 7.4. Variant M2 shows a 4-fold higher k_{cat} as also indicated in Table 2 using the crude cell extract, validating the characterization procedure with crude cell lysate. Furthermore, an increased Kₘ from 0.7 mM (wild type PpADI) to 1.2 mM (M1) and 2.5 mM (M2) was found.

### Relative pH profile of wild type and mutant

The relative pH profile (Figure 3) of wild type PpADI and M1 (H404R) shows an identical pH optimum at pH 6.5. Compared to wild type PpADI, variant M2 (K5T D44E H404R) shows a pH optimum at pH 7.0 which is shifted remarkably (0.5 pH units). Both M1 and M2 show decreased specific activities at pH 6.5, and increased specific activity at pH 7.5. At pH 7.0, the specific activity of M2 exceeds that of wild type PaADI (data not shown).

**Table 1. Mutations of variants from epPCR**

| Mutants | Mutations |
|---|---|
| **M1** | 404 His (CAC)→Arg (CGT) |
| **M2** | 5 Lys (AAA) →Thr (ACA) 44 Asp (GAT) →Glu (GAA) 404 His (CAC)→Arg (CGT) |
| | 3 Ala (GCT) →Ala (GCC) 207 Phe(TTC) →Phe (TTT) 278 His (CAC) →His (CAT) 291 Val (GTC) →Ala (GCC) 404 His |
| 15-G10-2 | (CAC)→Arg (CGT) 147 Tyr (TAC) →His (CAC) 326 Ile (ATC) →Thr (ACC) |
| 2-H4-2 | 342 Thr (ACC) →Thr (ACT) 404 His (CAC)→Arg (CGT) 113 Gly (GGC) →Ser (AGC) 128 Ala (GCC) →Thr (ACC) |
| 1-G11-1 | 292 Ser (ACG) →Thr (TCG) 404 His (CAC)→Arg (CGT) 3 Ala (GCC) →Ala (GCT) 128 Ala (GCC) →Thr (ACC) |
| 9-B3-1 | 362 Ala (GCG) →Ala (GCA) 404 His (CAC)→Arg (CGT) 128 Ala (GCC) →Val (GTC) 150 His (CAC) →Arg (CGC) 404 His |
| 21-DS-1 | (CAC)→Arg (CGT) |
| 24-E10-1 | 30 Lys (CAA) →Glu (CGA) 404 His (CAC)→Arg (CGT) |

**Table 2. Activity of preferred ADI variants towards arginine, determined using the cuvette format citrulline colorimetric assay**

| ADI variants | Absolute values not normalized [ADI] | | | | Normalized [ADI]* | | | | Activity at pH 7.4/ activity at pH 6.4 |
|---|---|---|---|---|---|---|---|---|---|
| | Citrulline production (mM) | | Relative to activity at pH 7.4 of WT | | Citrulline production (mM/mg) | | Relative to activity at pH 7.4 of WT | | |
| | pH 7.4 | pH 6.4 | pH 7.4 | pH 6.4 | pH 7.4 | pH 6.4 | pH 7.4 | pH 6.4 | |
| **WT M1 (H404R) M2 (K5T D44E H404R)** | 0.48 | 6.09 | 1.00 | 12.71 | 9.83 | 124.92 | 1.00 | 12.69 | 0.08 |
| | (0.04) 1.27 | (0.10) 3.29 | (0.15) 2.66 | (0.01) 6.86 | (0.74) 17.80 | (1.98) 45.95 | (0.15) 1.81 | (0.01) 4.67 | (0.01) 0.39 |
| | (0.04) 3.41 | (0.06) 3.43 | (0.04) 7.13 | (0.01) 7.17 | (0.49) 38.99 | (0.87) 39.20 | (0.06) 3.96 | (0.02) 3.98 | (0.02) 0.99 |
| | (0.07) 4.54 | (0.03) 9.94 | (0.01) 9.48 | (0.01) 20.75 | (0.79) 24.89 | (0.31) 54.50 | (0.02) 2.53 | (0.02) 5.54 | (0.03) 0.46 |
| *9-B3-1* | (0.08) 3.43 | (0.20) 6.80 | (0.01) 7.15 | (0.01) 14.20 | (0.45) 25.68 | (1.80) 50.95 | (0.03) 2.61 | (0.03) 5.18 | (0.02) 0.50 |
| *21-D5-1* | (0.36) 1.74 | (0.31) 6.25 | (0.03) 3.64 | (0.01) 13.06 | (2.68) 13.11 | (2.36) 47.02 | (0.07) 1.33 | (0.02) 4.78 | (0.01) 0.28 |
| 15-G10-2 | (0.07) 1.97 | (0.06) 3.41 | (0.03) 4.12 | (0.01) 7.11 | (0.53) 12.50 | (0.47) 21.58 | (0.09) 1.27 | (0.02) 2.19 | (0.01) 0.58 |
| 2-H4-2 | (0.01) 2.40 | (0.01) 2.81 | (0.02) 5.01 | (0.01) 5.86 | (0.07) 24.91 | (0.09) 29.12 | (0.06) 2.53 | (0.04) 2.96 | (0.01) 0.85 |
| 1-G11-1 | (0.02) 1.35 | (0.03) 3.03 | (0.02) 2.82 | (0.01) 6.32 | (0.20) 14.62 | (0.33) 32.82 | (0.03) 1.49 | (0.03) 3.34 | (0.02) 0.45 |
| 24-E10-1 | (0.06) | (0.05) | (0.04) | (0.01) | (0.62) | (0.52) | (0.08) | (0.03) | (0.02) |

| | | | | | | | | | |
|---|---|---|---|---|---|---|---|---|---|
| * Based on quantification of Agilent 2100 Bioanalyzer § Standard error of triplicate measurements is indicated in bracket | | | | | | | | | |

**Table 2 (cont.) Additional preferred ADI variants with Kₘ improvement**

| Variant | Mutation | Relative Kₘ |
|---|---|---|
| 1-H7 (parent)* | K5T, D44E, A128T*, H404R | 1.0 |
| 8-B7 | K5T, **Asp38His**, D44E, A128T, H404R | 0.5 |
| 6-B6 | K5T, D44E, A128T, **Glu296Lys,** H404R | 0.1 |

| | | |
|---|---|---|
| * A128T is responsible for expression improvement. | | |

### References

1. Ni Y, Schwaneberg U, Sun ZH. Arginine deiminase, a potential anti-tumor drug. Cancer Lett, 2008; 261 (1): 1-1 1.
2. Park IS, Kang SW, Shin YJ, et al. Arginine deiminase: a potential inhibitor of angiogenesis and tumour growth. Br J Cancer, 2003; B9(5): 907-14.
3. Izzo F, Marra P, Beneduce G, et al. Pegylated arginine deiminase treatment of patients with unresectable hepatocellular carcinoma: Results from phase I/II studies. J Clin Oncol. 2004; 22(10): 181 5-22.
4. Gong H, Zölzer F, von Recklinghausen G, Havers W, Schweigerer L. Arginine deiminase inhibits proliferation of human leukemia cells more potently than asparaginase by inducing cell cycle arrest and apoptosis. Leukemia, 2000; 14(5): 826-9.
5. Shen W, Shen WC. Drug evaluation: ADI-PEG-20 - a PEGylated arginine deiminase for arginine auxotrophic cancers. Curr Opin in Mol Ther, 2006; 8(3): 240-8.
6. Ensor CM, Holtsberg W, Bomalaski JS, Clark MA. Pegylated arginine deiminase (ADI-SS PEG (20,000) (mw)) inhibits human melanomas and hepatocellular carcinomas in vitro and in vivo. Cancer Res, 2002; 62(19): 5443-50.
7. Holtsberg RN, Ensor CM, Steiner MR, Bornalaski JS, Clark MA. Poly(ethylene glycol) (PEG) conjugated arginine deiminase: effects of PEG formulations on its pharmacological properties. J Controlled Release. 2002; 80(1-3): 259-71.
8. Müller HJ, Boos J. Use of L-asparaginase in childhood all. Crit Rev Oncol Hematol. 1998; 28(2): 97-1 13.
9. Tomschy A. Brugger R. Lehmann M, et al. Engineering of phytase for improved activity at low pH. Appl Environ Microbiol, 2002; 68(4): 1907-13.
10. Turunen O. Janis J, Fenel F, Leisola M. Engineering the thermotolerance and pH optimum of family 11 xylanases by site-directed mutagenesis. Methods Enzymol, 2004; 388: 156-67.
11. Fang TY, Ford C. Protein engineering of Aspergillus awamori glucoamylase to increase its pH optimum. Protein Eng, 1998; 11(5): 383-8.
12. Bessler C, Schmitt J, Maurer KH, Schmid RD. Directed evolution of a bacterial alpha-araylase: Toward enhanced pH-performance and higher specific activity. Protein Sci, 2003; 12(10): 2141-9.
13. Hirata A, Adachi M, Utsumi S. Mikami B. Engineering of the pH optimum of Bacillus cereus beta-amylase: Conversion of the pH optimum from a bacterial type to a higher-plant type. Biochemistry (Mosc), 2004; 43(39): 12523-31.
14. Sakoda H, Imanaka T. Cloning and sequencing of the gene coding for alcohol-dehydrogenase of Bacillus stearothermophilus and rational shift of the optimum pH. J Bacteriol, 1992; 174(4): 1397-402.
15. Galkin A, Lu XF, Dunaway-Mariano D, Herzberg O. Crystal structures representing the Michaelis complex and the thiouronium reaction intermediate of Pseudomonas aeruginosa arginine deiminase. J Biol Chem, 2005; 280(40): 3408e7.
16. Wang WY, Malcolm BA. Two-stage PCR protocol allowing introduction of multiple mutations, deletions and insertions using QulkChange™ site-directed mutagenesis. Biotechniques, 1999; 26(4): 680-2.
17. Miyazaki K, Takenouchi M. Creating random mutagenesis libraries using megaprimer PCR of whole plasmid. BioTechniques, 2002; 33(5): 1033-8.
18. Studier FW. Protein production by auto-induction in high-density shaking cultures. Protein Expression Purif, 2005; 41(1): 207-34.
19. Archibald RM. Detemination of citrulline and allantoin and demonstration of citrulline in blood plasma. J Biol Chem, 1944; 156(1): 121-42.
20. Boyde TRC, Rahmatullah M. Optimization of conditions for the calorimetric determination of citrulline, using diacetyl monoxime. Anal Biochem, 1980; 107(2): 424-31.
21. Guex N, Peitsch MC. SWISS-MODEL and the Swiss-PdbViewer: an environment for comparative protein modeling. Electrophoresis, 1 997; 18(15): 2714-23.
22. van Gunsteren WF, Billeter SR, Eising AA, et al. Bimolecular simulation: the gromos 96 manual and user guide ETH Zürich; 1996.
23. Wong TS, Arnold FH, Schwaneberg U. Laboratory evolution of cytochrome P450BM-3 monooxygenase for organic cosolvents. Biotechnol Bioeng, 2004; 85(3): 351 -8.
24. Glieder A, Farinas ET, Arnold FH. Laboratory evolution of a soluble, self-sufficient, highly active alkane hydroxylase. Nat Biotechnol, 2002; 20(11): 1135-9.
25. Tee KL, Dmytrenko O, Otto K, Schmid A, Schwaneberg U. A p-nitrothiophenolate screening system for the directed evolution of a two-component epoxygenase (StyAB). J Mol Catal B: Enzym, 2008; 50(2-4): 121-7.
26. Zhu ZW. Momeu C, Zakhartsev M, Schwaneberg U. Making glucose oxidase fit for biofuel cell applications by directed protein evolution. Biosens Bioelectron, 2006; 21(15): 2046-51.
27. Takaku H, Matsumoto M, Misawa S, Miyazaki K. Antitumor-activity of arginine deiminase from Mycoplasma arginini and its growth-Inhibitory mechanism. Jap J Cancer Res, 1995; 86(9): 840-6.
28. Chuang VTG, Kragh-Hansen U, Otagiri M. Pharmaceutical strategies utilizing recombinant human serum albumin. Pharm Res, 2002; 19(5): 569-77.
29. Lu XF, Li L, Wu R, et al. Kinetic analysis of Pseudomonas aeruginosa arginine deiminase mutants and alternate substrates provides insight into structural determinants of function. Biochemistry (Mosc), 2006; 45(4): 11 62-72.
30. Li L, Li ZM, Wang CH, et al. The electrostatic driving force for nucleophilic catalysis in L-arginine deiminase: a combined experimental and theoretical study. Biochemistry (Mosc), 2008; 47(16): 4721 -32.
31. Stemmer WPC. Rapid evolution of a protein in vitro by DNA shuffling. Nature, 1994; 370(6488): 389-91.

## Claims

1. Modified arginine deiminase, comprising
a) at least one modified amino acid in close proximity to loop 4 of said arginine deiminase and in close proximity to the active-site cysteine of said arginine deiminase, wherein said modified amino acid has a positively charged side chain at a physiological pH; and
b) optionally, at least one modified amino acid in loop 1 of said arginine deiminase, wherein said modified amino acid is modified to reduce the distance with an amino acid in said loop 4 in order to favor the formation of a hydrogen bond,
and wherein said modified arginine deiminase exhibits an increased activity at a higher pH, compared to a non-modified arginine deiminase.

2. The modified arginine deiminase according to claim 1, comprising one modified amino acid in close proximity to loop 4 of said arginine deiminase and in close proximity to the active-site cysteine of said arginine deiminase, wherein said modified amino acid has a positively charged side chain at a physiological pH, and wherein said modified amino acid is preferably a basic amino acid, such as, for example, arginine.

3. The modified arginine deiminase according to any of claims 1 or 2, comprising one modified amino acid in close proximity to loop 4 of said arginine deiminase and in close proximity to the active-site cysteine of said arginine deiminase, wherein said modified amino acid has a positively charged side chain at a physiological pH and one modified amino acid in loop 1 of said arginine deiminase, wherein said modified amino acid is modified to reduce the distance with an amino acid in said loop 4 in order to favor the formation of a hydrogen bond, wherein said modified amino acid preferably has an elongated side chain, such as, for example, glutamic acid.

4. The modified arginine deiminase according to any of claims 1 to 3, which is derived from *Pseudomonas plecoglossicida* ADI, *Pseudomonas aeruginosa* ADI, *Mycoplasma arginini* ADI, *Mycoplasma arthritidis* ADI, or *Mycoplasma hominis* ADI, and preferably is selected from *Pseudomonas plecoglossicida* ADI H404R or *Pseudomonas plecoglossicida* ADI H404R D44E.

5. The modified arginine deiminase according to any of claims 1 to 4, wherein said arginine deiminase exhibits a decreased specific activity for a conversion of arginine to citrulline at pH 6.5, and increased specific activity at least one of pH 7.0; pH 7.4, or pH 7.5, and preferably exhibits a pH optimum at at least one of pH 7.0; pH 7.4, or pH 7.5, and/or exhibits a 4-fold higher k_{cat} for a conversion of arginine to citrulline at a pH of 7.4, compared to a non-modified arginine deiminase.

6. The modified arginine deiminase according to any of claims 1 to 5, wherein said arginine deiminase comprises at least one additional modified amino acid and/or is pegylated, wherein said at least one modified amino acid and/or pegylation does not substantially interfere with the increased activity of said arginine deiminase at said higher pH.

7. A nucleic acid, encoding for a modified arginine deiminase according to any of claims 1 to 6, wherein said nucleic acid preferably is included in an expression cassette or vector.

8. A method for modifying the pH optimum of the activity of an arginine deiminase, comprising the steps of
a) modifying at least one amino acid in close proximity to loop 4 of said arginine deiminase and in close proximity to the active-site cysteine of said arginine deiminase, wherein said modification of said amino acid provides a positively charged side chain of said amino acid at a physiological pH; and
b) optionally, modifying at least one amino acid in loop 1 of said arginine deiminase, wherein said amino acid is modified to reduce the distance with an amino acid in said loop 4 in order to favor the formation of a hydrogen bond,
and wherein said modifying provides an increased activity of said modified arginine deiminase at a higher pH, compared to a non-modified arginine deiminase.

9. The method according to claim 8, wherein said modification provides a basic amino acid, such as, for example, arginine, and/or wherein said modification further provides an amino acid having an elongated side chain, such as, for example, glutamic acid.

10. The method according to according to any of claims 8 or 9, wherein said arginine deiminase to be modified is derived from *Pseudomonas plecoglossicida* ADI, *Pseudomonas aeruginosa* ADI, *Mycoplasma arginini* ADI, *Mycoplasma arthritidis* ADI, or *Mycoplasma hominis* ADI, wherein preferably said modification is selected from H404R or H404R and D44E in *Pseudomonas plecoglossicida* ADI.

11. The method according to according to any of claims 8 to 16, wherein said arginine deiminase after modification exhibits a decreased specific activity for a conversion of arginine to citrulline at pH 6.5, and increased specific activity at least one of pH 7.0; pH 7.4, or pH 7.5, and preferably exhibits a pH optimum at at least one of pH 7.0; pH 7.4, or pH 7.5, and/or after modification exhibits a 4-fold higher k_{cat} for a conversion of arginine to citrulline at a pH of 7.4, compared to a non-modified arginine deiminase.

12. The method according to according to any of claims 8 to 11, wherein said method further comprises modifying of at least one additional amino acid, pegylation and/or fusion to a second polypeptide, wherein said modifying of said at least one modified amino acid, pegylation and/or fusion does not substantially interfere with the increased activity of said arginine deiminase at said higher pH.

13. The method according to according to any of claims 8 to 12, wherein said method further comprises at least one step selected from the group consisting of mutating said arginine deiminase, such as, for example, by error-prone PCR, a citrulline detection assay, and a colorimetric detection assay.

14. A pharmaceutical composition, comprising a modified arginine deiminase according to any of claims 1 to 6 or an arginine deiminase modified according to any of claims 8 to 13, together with a pharmaceutically acceptable carrier.

15. Use of a pharmaceutical composition according to claim 14, or a modified arginine deiminase according to any of claims 1 to 6 or an arginine deiminase modified according to any of claims 8 to 13 for the treatment of arginine-auxotrophic cancers, such as hepatocellular carcinomas and melanomas, or for the treatment of tumor-induced neovascularization in a cancer, such as hepatocellular carcinomas and melanomas.
